(19) **Europäisches Patentamt**
**European Patent Office**
**Office européen des brevets**

(11) **EP 1 542 268 A1**

(12) **EUROPEAN PATENT APPLICATION**
published in accordance with Art. 158(3) EPC

(43) Date of publication:
**15.06.2005 Bulletin 2005/24**

(51) Int Cl.7: **H01L 21/3065**

(21) Application number: **03764209.7**

(22) Date of filing: **16.07.2003**

(86) International application number:
**PCT/JP2003/009023**

(87) International publication number:
**WO 2004/008515 (22.01.2004 Gazette 2004/04)**

(84) Designated Contracting States:
**AT BE BG CH CY CZ DE DK EE ES FI FR GB GR HU IE IT LI LU MC NL PT RO SE SI SK TR**

(30) Priority: **17.07.2002 JP 2002208604**

(71) Applicant: **Zeon Corporation**
**Tokyo 100-0005 (JP)**

(72) Inventors:
• **YAMADA, Toshiro**
  **Chiyoda-ku, Tokyo 100-0005 (JP)**
• **SUGIMOTO, Tatsuya**
  **Chiyoda-ku, Tokyo 100-0005 (JP)**

(74) Representative: **Albrecht, Thomas, Dr.**
**Kraus & Weisert,**
**Thomas-Wimmer-Ring 15**
**80539 München (DE)**

(54) **METHOD OF DRY ETCHING, DRY ETCHING GAS AND PROCESS FOR PRODUCING PERFLUORO-2-PENTYNE**

(57)    A dry etching method wherein a resist film is irradiated with radiation having a wavelength of not more than 195 nm to form a resist pattern having a minimum line width of not more than 200 nm, and the substrate having the resist pattern formed thereon is subjected to dry etching using a fluorine-containing compound having 4 to 6 carbon atoms and at least one unsaturated bond as an etching gas. As the fluorine-containing compound, perfuloro-2-pentyne, perfuloro-2-butyne, nonafluoro-2-pentene and perfluoro-2-pentene are preferably used. Perfuloro-2-pentyne is produced by a process wherein a 1,1,1-trihalo-2,2,2-trifluoroethane is allowed to react with pentafluoropropionaldehyde to give a 2-halo-1,1,1,4,4,5,5,5-octafluoro-2-pentene, and the thus-produced halo-octafluoro-2-pentene is dehydrohalogenated.

**Description**

Technical Field

This invention relates to a method of dry etching, a dry etching gas, and a process for producing perfluoro-2-pentyne. More particularly, this invention relates to a method of dry etching wherein an etching rate is high, a selectivity for substrate is high, and a fine pattern having a minimum line width of not more than 200 nm can be formed on a silicon substrate at an enhanced stability; a dry etching gas; and a process for producing perfluoro-2-pentyne used for the dry etching.

Background Art

With the advance in high integration and high performance of integrated circuits of semiconductor devices such as VLSI (very large scale integrated circuit) and ULSI (ultra large scale integrated circuit) in recent years, technical demands for a dry etching gas used in the production process of these semiconductor devices are becoming increasingly strict. As the dry etching gas, saturated fluorocarbon gases such as carbon tetrafluoride and perfluorocyclobutane have heretofore been widely used. However, it is said that saturated fluorocarbon gases have a long life in the air, namely, a life of several-thousands years, and exert a considerable influence upon the global warming. Therefore, various fluorine-containing compounds have been developed as alternatives for saturated fluorocarbon gases.

Fluorine-containing compounds having an unsaturated bond are disclosed in Japanese Unexamined Patent Publication H9-191002. It is reported in this patent publication that evaluation of etching was conducted on a resist pattern formed with radiation having a wavelength of 300 nm by a KrF excima laser lithography, and good results were obtained. In Japanese Unexamined Patent Publication 2002-184768, it is reported that evaluation of etching was conducted on a resist pattern formed by lithography using deep UV, namely, ultraviolet rays of a wavelength of 200 to 300 nm, and good results were obtained.

For the exposure to radiation with a wavelength of at least 200 nm, for example, irradiation using a KrF with a wavelength of 248 nm, a resist formed from a high molecular weight compound having an aromatic ring structure has heretofore been widely used. This is because a resist formed from a high molecular weight compound having an aromatic ring structure exhibits a high resistance for dry etching, and, at an etching step after light exposure and development, a high etching selectivity for silicon oxide substrate can be obtained. However, a resist pattern having a minimum line width of 210 nm can be obtained at the utmost with a KrF (248 nm) excima laser used for 256 megabit DRAM. With further advance in integration of integrated circuits in a semiconductor device, an ArF (193 nm) excima laser or other light sources of shorter wavelength should be used for the production of DRAM with 1 gigabit or more. However, in the case where a high molecular weight compound having an aromatic ring structure is used, a fine pattern cannot be formed when radiation with a wavelength of 200 nm or less such as ArF (193 nm) laser or X-rays is used, because radiation is absorbed to an undue extent by an aromatic ring.

Now high molecular weight compounds having an alicyclic structure and alkali-soluble methacrylic acid copolymers are being examined for the formation of a resist for radiation with a wavelength of 195 nm or less such as ArF and X-rays. However, these high molecular weight compounds do not contain or contain only a minor proportion of an aromatic ring structure, and therefore, a problem arises at an etching step after light exposure and development, in that an unexposed area of resist tends to be undesirably etched depending upon the particular kind of etching gas, and thus, the etching selectivity for silicon oxide is low. For example, in the case when perfluoropropene, which is said to be useful for a resist for radiation with a wavelength of more than 195 nm, is used for dry etching of a silicon oxide film, a protective coating such as polysilicon as well as an unexposed area of a photoresist tend to be undesirably etched, and thus, the etching selectivity for silicon oxide is not high and a fine pattern is difficult to form.

Especially, with a great reduction in line width of a resist pattern, for example, in the order of 200, 180, 130 and 100 nm, a resist formed on a substrate represented by silicon oxide film becomes thinner, and therefore, a high etching selectivity becomes more difficult to attain.

Disclosure of the Invention

An object of the present invention is to provide a method of dry etching by which a high etching rate and a high etching selectivity can be attained and a fine resist pattern with a minimum line width of 200 nm or less can be formed with good stability, even for a resist having poor resistance to dry etching using a light source with a short wavelength of 195 nm or less.

Another object of the present invention is to provide a dry etching gas by which a high etching rate and a high etching selectivity can be attained and a fine resist pattern with a minimum line width of 200 nm or less can be formed with good stability, even for a resist having poor resistance to dry etching using a light source with a short wavelength of 195 nm or less.

A further object of the present invention is to provide a process for producing perfluoro-2-pentyne which is suitable for use as the above-mentioned dry etching gas.

The inventors made an extensive research for achieving the above-mentioned objects, and found that, in the case when dry etching is carried out using a fluorine-containing compound having 4 to 6 carbon atoms and at least one unsaturated bond as an etching gas, a high etching selectivity can be attained without reduction of etching rate, even for a resist having poor resistance to dry etching. Based on this finding, the present invention has been completed.

Thus, in one aspect of the present invention, there is provided a dry etching method characterized in that a resist film formed on a substrate is irradiated with radiation having a wavelength of not more than 195 nm to form a resist pattern having a minimum line width of not more than 200 nm, and the substrate having the resist pattern formed thereon is subjected to dry etching using a fluorine-containing compound having 4 to 6 carbon atoms and at least one unsaturated bond as an etching gas.

In another aspect of the present invention, there is provided a dry etching gas comprised of a fluorine-containing compound having 4 to 6 carbon atoms and at least one unsaturated bond, and used for dry etching for a resist film forming a resist pattern having a minimum line width of not more than 200 nm at irradiation with radiation having a wavelength of not more than 195 nm.

In a further aspect of the present invention, there is provided a process for producing perfluoro-2-pentyne characterized in that a 1,1,1-trihalo-2,2,2-trifluoroethane is allowed to react with pentafluoropropionaldehyde to give a 2-halo-1,1,1,4,4,5,5,5-octafluoro-2-pentene, and the thus-produced 2-halo-1,1,1,4,4,5,5,5-octafluoro-2-pentene is dehydrohalogenated.

Best Mode for Carrying Out the Invention

In the dry etching method of the present invention, a resist film formed on a substrate is irradiated with radiation having a wavelength of not more than 195 nm to form a resist pattern having a minimum line width of not more than 200 nm, and then, the substrate having the resist pattern thus-formed thereon is subjected to dry etching using a fluorine-containing compound having 4 to 6 carbon atoms and at least one unsaturated bond as an etching gas. By this method, the etching of a substrate can be effectively carried out and thus, a fine pattern of a minimum line width of not more than 200 nm, preferably not more than 180 nm, more preferably not more than 150 nm and especially preferably not more than 140 nm can be easily formed with a high precision. By the term "minimum line width" as used herein, we mean broadly those which include, for example, a diameter of contact holes in the case when the resist pattern is contact holes.

If a fluorine-containing compound having at least one unsaturated bond, but having three or less carbon atoms, is used as a dry etching gas, the selectivity in dry etching tends to be reduced. A fluorine-containing compound having at least one unsaturated bond but having at least 7 carbon atoms is usually difficult to use as a dry etching because it has a high boiling point.

The resist film formed on a substrate is not particularly limited, but is preferably formed from a high molecular weight compound containing 0% to 10% by weight of repeating units having an aromatic ring structure. The content of repeating units having an aromatic ring structure in the high molecular weight compound constituting the resist film is more preferably in the range of 0% to 5% by weight, especially 0% to 2% by weight. When the content of repeating units having an aromatic ring structure in the high molecular weight compound constituting the resist film is in the range of 0% to 10% by weight, transparency to radiation with a wavelength of not more than 195 nm can be obtained and a desired resist pattern having a minimum line width of not more than 200 nm can be formed.

As specific examples of the high molecular weight compound containing 0% to 10% by weight of repeating units having an aromatic ring structure, there can be mentioned a partially hydrogenated resin product of a p-hydroxystyrene/t-butyl methacrylate copolymer, a partially hydrogenated resin product of a p-hydroxystyrene/4-ethenylphenoxyacetic acid 1-alkylcyclohexyl ester copolymer, an isobornyl methacrylate/methyl methacrylate/t-butyl methacrylate/methacrylic acid quaternary polymer, an adamantyl methacrylate/t-butyl methacrylate copolymer resin, a valerolactone metacrylic acid ester/2-alkyladamantyl methacrylate/hydroxyadamantyl methacrylate terpolymer resin, a t-butyl methacrylate/vinyl alkyl ether/maleic anhydride terpolymer resin, a norbornenecarboxylic acid/t-butyl norbornenecarboxylate/alkylnorbornene/maleic anhydride quaternary polymer resin, a norbornenecarboxylic acid/t-butyl norbornenecarboxylate/alkylnorbornene terpolymer resin, a hydrogenated product resin of a metathesis ring-open copolymer of an alkyl norbornenecarboxylate with t-butyl tricyclodecenecarboxylate, and a hydrogenated product resin of a metathesis ring-open copolymer of a norbornene t-butylcarboxylic acid ester with tricyclodecenecarboxylic acid alkyl ester.

The procedure for forming a resist film on a substrate is not particularly limited and the conventional procedure may be adopted. For example, a solution of a resist composition is applied onto a substrate by spin coating or other coating means, and then a solvent is removed by evaporation from the coating solution to be thereby dried. According to the need, the dried coating is pre-baked. In the solution of a resist composition, an acid generator such as an onium salt, diazomethane derivatives and glyoxime derivatives can be incorporated according to the need.

The radiation used in the present invention has a wavelength of not more than 195 nm, preferably not more than 170 nm especially preferably not more than 150 nm. The radiation is not particularly limited provided that it has a wavelength of not more than 195 nm. As specific examples of the radiation used, there can be mentioned ArF excima laser (193 nm), $F_2$ excima laser (157 nm), $Kr_2$ excima laser (146 nm), KrAr excima laser (134 nm), $AR_2$ excima laser (126 nm), vacuum ultraviolet rays (not more than 170 nm), electron beam (EB) and X rays.

The procedure for forming a resist pattern is also not particularly limited. For example, a resist film is masked and irradiated with radiation according to the pattern. Then the irradiated resist is post-baked according to the need, and development is carried out using an aqueous alkali such as an aqueous tetramethylammonium hydroxide solution to form a resist pattern, followed by dry etching.

In the dry etching method of the present invention, a fluorine-containing compound having 4 to 6 carbon atoms and at least one unsaturated bond is used as a dry etching gas. By the term "fluorine-containing compound" as used herein, we mean a compound composed of carbon and fluorine or a compound composed of carbon, hydrogen and fluorine. The unsaturated bond may be either a double bond or a triple bond. The fluorine-containing compound may have both a double bond and a triple bond.

As specific examples of the fluorine-containing compound having a triple bond, there can be mentioned perfuoro-1-butyne, perfuoro-2-butyne, perfuoro-1-pentyne, perfuoro-2-pentyne, perfuoro-1,3-pentadiyne, perfuoro-1,4-pentadiyne, perfuoro-1-hexyne, perfuoro-2-hexyne, perfuoro-3-hexyne, perfuoro-1,3-hexadiyne, perfuoro-1,4-hexadiyne, perfuoro-1,5-hexadiyne and perfuoro-2,4-hexadiyne.

As specific examples of the fluorine-containing compound having a double bond, there can be mentioned perfuoro-1-butene, perfuoro-2-butene, perfuoro-1,3-butadiene, perfuoro-1-pentene, perfuoro-2-pentene, perfuoro-1,3-pentadiene, perfuoro-1,4-pentadiene, perfuoro-2-methyl-1,3-butadiene, octafluorocyclopentene, hexafluorocyclopentadiene, perfluoro-1-hexene, perfluoro-2-hexene, perfluoro-3-hexene, perfluoro-1,3-hexadiene, perfluoro-1,4-hexadiene, perfluoro-1,5-hexadiene, perfluoro-2,4-hexadiene, perfluoro-1,3,5-hexatriene, perfluoro-1,3-cyclohexadiene, perfluoro-1,4-cyclohexadiene, perfluorobenzene, 1,1,1,2,4,4,4-heptafluoro-2-butene, 1,3,3,4,4-pentafluorocyclobutene, 1,2,3,4,4-pentafluorocyclobutene, 1,1,1,2,4,4,5,5,5-nonafluoro-2-pentene, 1,1,1,3,4,4,5,5,5-nonafluoro-2-pentene, 1,1,1,2,3,4,5,5,5-nonafluoro-2-pentene, 1,3,3,4,4,5,5-heptafluorocyclopentene, 1,2,3,4,4,5,5-heptafluorocyclopentene, 1,2,3,3,4,5,5-heptafluorocyclopentene, 1,1,1,3,4,4,5,5,6,6,6-undecafluoro-2-hexene, 1,1,1,2,4,4,5,5,6,6,6-undecafluoro-2-hexene, 1,1,1,2,2,4,5,5,6,6,6-undecafluoro-3-hexene and 1,1,1,2,2,3,5,5,6,6,6-undecafluoro-3-hexene.

As specific examples of the fluorine-containing compound having both of a double bond and a triple bond, there can be mentioned perfuoro-1-buten-3-yne, perfuoro-1-penten-3-yne, perfuoro-1-penten-4-yne, perfuoro-3-penten-1-yne, perfuoro-2-methyl-1-buten-3-yne, perfuoro-1-cyclohexen-3-yne and perfuoro-1-cyclohexen-4-yne.

The above-mentioned fluorine-containing compound having at least one unsaturated bond may be used either alone or as a combination of at least two thereof as a dry etching gas. Of the above-recited fluorine-containing compounds, perfluoro-2-butyne, perfluoro-2-pentyne, perfluoro-2-hexyne, perfluoro-3-hexyne, 1,1,1,2,4,4,5,5,5-nonafluoro-2-pentene, 1,1,1,3,4,4,5,5,5-nonafluoro-2-pentene and perfuoro-2-pentene are preferable because of high stability. 1,1,1,2,4,4,5,5,5-nonafluoro-2-pentene, 1,1,1,3,4,4,5,5,5-nonafluoro-2-pentene and perfuoro-2-pentene are especially preferable. Perfluoro-2-pentyne and perfluoro-2-butyne are preferable in view of high etching rate and high etching selectivity. Perfluoro-2-pentyne is especially preferable.

The fluorine-containing compound having 4 to 6 carbon atoms and at least one unsaturated bond preferably has a purity of at least 90% by volume, more preferably at least 99% by volume and especially preferably at least 99.9% by volume. If the fluorine-containing compound has a purity of lower than 90% by volume, the etching rate and the etching selectivity are liable to be poor.

A material to be etched includes, for example, silicon oxide, silicon nitride, aluminum, tungsten, molybdenum, tantalum, titanium, chromium, chromium oxide and gold. As specific examples of the substrate to be etched, there can be mentioned silicon wafer having silicon oxide film or aluminum film. The size of silicon wafer is not particularly limited, but the silicon wafer usually has a size of 8 inches or 12 inches.

At a dry etching step, etching is carried out preferably under irradiation with a plasma. The plasma preferably has a density of at least $10^{10}$ ions/cm$^3$, more preferably in the range of $10^{10}$ to $10^{13}$ ions/cm$^3$. When the plasma density is in the range of $10^{10}$ to $10^{13}$ ions/cm$^3$, higher etching rate and higher etching selectivity can be obtained and a fine pattern can be formed. As examples of an apparatus for generating plasma, there can be mentioned those which are of a helicon wave type, a high-frequency induction type, a parallel flat plate type and a magnetron type. Of these, a helicon wave type apparatus and a high-frequency induction type apparatus are preferable because a plasma having a high density region can be easily generated.

At a dry etching step, the fluorine-containing compound having 4 to 6 carbon atoms and at least one unsaturated bond is preferably introduced into an apparatus vacuumed to an extent such that the inner pressure reaches a pressure in the range of 0.0013 to 1,300 Pa, more preferably 0.13 to 1.3 Pa. The temperature which the substrate to be dry-etched reaches during the dry etching step is preferably in the range of 0 to 300°C, more preferably 60 to 250°C and especially preferably 80 to 200°C. The treating time for the dry etching is usually in the range of 10 seconds to 10 minutes. However, etching can be effected at a high etching rate in the dry etching method of the present invention, and therefore, the dry etching can be carried out for a treating time of 10 seconds to 3 minutes with a high productivity.

At a dry etching step, other gas can be additionally incorporated in the etching apparatus. The gas used in combination with the fluorine-containing compound includes, for example, rare gases such as helium, neon, argon, krypton and xenon; and oxygen and oxygen-containing gases such as carbon monoxide and carbon dioxide. Of these, oxygen is preferably used in combination with the fluorine-containing compound because oxygen has a dissociation accelerating action for an etching gas.

The dry etching gas used in the present invention is comprised of a fluorine-containing compound having 4 to 6 carbon atoms and at least one unsaturated bond, with which dry etching is carried out for a resist pattern having a minimum line width of not more than 200 nm formed by irradiation with radiation with a wavelength of not more than 195 nm. As the fluorine-containing gas having 4 to 6 carbon atoms, various gases can be used as mentioned above. Perfluoro-2-pentyne is especially preferably used because high etching rate and high etching selectivity can be obtained.

In the process for producing perfluoro-2-pentyne according to the present invention, a 1,1,1-trihalo-2,2,2-trifluoroethane is allowed to react with pentafluoropropionaldehyde to give a 2-halo-1,1,1,4,4,5,5,5-octafluoro-2-pentene, and the thus-produced 2-halo-1,1,1,4,4,5,5,5-octafluoro-2-pentene is dehydrohalogenated to give perfluoro-2-pentyne.

The conditions for allowing a 1,1,1-trihalo-2,2,2-trifluoroethane to react with pentafluoropropionaldehyde are not particularly limited. For example, pentafluoropropionaldehyde hydrate is treated with a dehydrating agent such as phosphorus pentoxide under heated conditions to be thereby dehydrated to give pentafluoropropionaldehyde, and the pentafluoropropionaldehyde is blown in a cooled polar solvent such as dimethylformamide to give a solution. To the thus-obtained solution, a 1,1,1-trihalo-2,2,2-trifluoroethane and powdery zinc oxide were added, and the mixture is heated to give a 2-halo-1,1,1,4,4,5,5,5-octafluoro-2-pentene. The amount of pentafluoropropionaldehyde hydrate used is preferably in the range of 1 to 2 moles, more preferably 1.2 to 1.5 moles, per mole of a 1,1,1-trihalo-2,2,2-trifluoroethane to be reacted with a dehydrated product of the pentafluoropropionaldehyde hydrate. The amount of powdery zinc added is preferably in the range of 2 to 3 moles, more preferably 2.2 to 2.5 moles, per mole of the 1,1,1-trihalo-2,2,2-trifluoroethane. Powdery zinc oxide is preferably previously activated by treating with, for example, dilute aqueous hydrochloric acid or acetic anhydride prior to the addition to the pentafluoropropionaldehyde solution. A preferable example of the starting material, 1,1,1-trihalo- 2,2,2-trifluoroethane, is 1,1,1-trichloro-2,2,2-trifluoroethane. The reaction temperature is usually in the range of room temperature to 150°C, more preferably 50°C to 100°C. The reaction time is usually in the range of 1 to 20 hours, more preferably 3 to 10 hours.

An after-treatment for the above-mentioned reaction may be conventional. That is, the reaction is stopped by adding dilute hydrochloric acid to the reaction liquid, and the reaction product is extracted with an extract solvent such as ethyl acetate or diethyl ether, neutralized with a saturated aqueous sodium bicarbonate solution, and then dried over a desiccating agent such as sodium sulfate. The dried product is concentrated by a rotary evaporator to give a crude purified product. The crude purified product may be subjected to the dehydrohalogenation, or, may be distilled for purification and then subjected to the dehydrohalogenation.

Then a basic compound such as, for example, potassium hydroxide is added to the obtained 2-halo-1,1,1,4,4,5,5,5-octafluoro-2-pentene whereby dehydrohalogenation is effected to produce a triple bond, namely, give perfluoro-2-pentyne. More specifically, a pressure-resistant autoclave is charged with a basic compound and the 2-halo-1,1,1,4,4,5,5,5-octafluoro-2-pentene, and the content is heated, and a gaseous product produced by the reaction is collected in a cooled trap. Alternatively, a procedure utilizing a feature such that perfluoro-2-pentyne to be produced has a boiling point of 5°C can be adopted. That is, a reactor is previously charged with a basic compound, and, while the content was stirred, the 2-halo-1,1,1,4,4,5,5,5-octafluoro-2-pentene was dropwise added and a gaseous product produced by the reaction is collected in a cooled trap.

The amount of basic compound used is preferably in the range of 1 to 10 moles, more preferably 2 to 5 moles, per mole of the 2-halo-1,1,1,4,4,5,5,5-octafluoro-2-pentene. The reaction temperature is usually in the range of room temperature to 250°C, more preferably 50°C to 200°C.

The collected perfluoro-2-pentyne can be subjected to distillation for enhancing the purity.

The reaction scheme in the above-mentioned production process of the present invention is shown below, wherein X represents halogen selected from chlorine, bromine and iodine.

$$CF_3CX_3 + CF_3CF_2CHO + 2Zn$$

$$\rightarrow CF_3CX=CHCF_2CF_3 + ZnX_2 + ZnO$$

$$CF_3CX=CHCF_2\,CF_3 + KOH$$

$$\rightarrow CF_3C\equiv CCF_2\,CF_3 + KX + H_2O$$

According to the process for producing perfluoro-2-pentyne, perfluoro-2-pentyne having a high purity can be produced with an enhanced efficiency from a readily available raw material, as compared with heretofore proposed processes for producing perfluoro-2-pentyne, which include dechlorination of 2,3-dichloro-1,1,1,4,4,5,5,5-octafluoro-2-pentene by using zinc, isomerization of perfluoropentadiene, or dehydrofluorination of 1,1,1,2,3,4,4,5,5,5-decafluoropentane.

Examples

The invention will now be described more specifically by the following working examples that by no means limit the scope of the invention.

In the working examples, parts are by weight unless otherwise specified.

In examples and comparative examples, selectivity ratio in etching was calculated by the following equation.

Selectivity ratio etching = (etching rate for silicon oxide film)/(etching rate for resist)

Production Example 1

An autoclave made of Hastelloy was charged with 394 parts of commercially available palletized potassium hydroxide (purity: 85% by weight) and 300 parts of 1,1,1,2,3,4,4,5,5,5-decafluoropentane ("Vertrel™ XF" available from Du pont). The content was thoroughly stirred and a reaction was carried out at 200°C for 7.5 hours.

The autoclave was cooled, and then, in order for distilling and collecting the thus-produced reaction mixture, a condenser and a vacuum pump were connected to the autoclave. The reaction mixture was collected into the condenser, which was cooled under a reduced pressure by liquid nitrogen, to give 182.5 parts of a reaction product. Analysis of the reaction product by gas chromatography revealed that it contained perfluoro-2-pentyne (target product), 1,1,1,2,4,4,5,5,5-nonafluoro-2-pentene, 1,1,1,3,4,4,5,5,5-nonafluoro-2-pentene and a small amount of 1,1,1,2,3,4,4,5,5,5-decafluoropentane (raw material). The yield of the target product was 20.6% by weight based on the amount of the raw material charged. A great portion of the unreacted raw material was decomposed and polymerized, which remained within the autoclave, and thus, the reaction product contained only about 1% by weight of raw material.

The above-mentioned reaction procedure was repeated to collect the reaction products. 1,202 parts of the collected reaction products (content of perfluoro-2-pentyne: 31.96% by volume) was fractionated under normal pressure by using a KS type fractionating column with a theoretical plate number of 35. A cooling medium at the top of the fractionating column was maintained at a temperature of -5 to -10°C, and a trap for collecting the desired fraction was maintained at a temperature of -78°C. This fractionation gave 264 parts of a perfluoro-2-pentyne fraction (boiling point: 5°C) having a purity of 99.9% by volume.

Production Example 2

200 parts of commercially available perfluoro-2-butyne (available from SynQuest Laboratories, Inc., purity: 98% by volume) was fractionated by using a pressure distillation column (PMN-2507FF available from Toka Seiki K. K., theoretical plate number: 33) to give 123 parts of perfluoro-2-butyne having a purity of 99.9% by volume and a boiling point of -24°C.

Production Example 3

200 parts of commercially available perfluoro-2-pentene (available from SynQuest Laboratories, Inc., purity: 99% by weight) was fractionated under normal pressure by using a KS type fractionating column with a theoretical plate number of 35. A cooling medium at the top of the fractionating column was maintained at -5 to -10°C and a fraction was collected in a flask cooled by ice water. The fractionation gave 157 parts of perfluoro-2-pentene (boiling point: 26°C) having a purity of 99.9% by weight.

Example 1

Ten parts of a terpolymer comprised of 5-norbornene-2-methanol units, maleic anhydride units and t-butyl methacrylate units (copolymerization ratio: 1:1:1 by mole; molecular weight: 5,500) and 0.2 part of triphenylsulfonium trifluoromethane sulfonate as an acid generator were dissolved in 70 parts of propylene glycol monomethyl ether acetate, and the resulting solution was filtered through a filter having a pore diameter of 100 nm to give a resist solution.

A silicon substrate of an 8 inch diameter having a coating of silicon oxide film with a thickness of 2 $\mu$m was coated with the above-mentioned resist solution by a spin-coating procedure, and then, the thus-coated substrate was pre-baked at 120°C on a hot plate to form a resist film having a thickness of 450 nm. The resist film was exposed to light through a mask pattern by an ArF excima-laser aligner (wavelength: 193 nm). The light-exposed resist film was post-baked at 130°C, and then subjected to a developing treatment at 25°C for 60 seconds with an aqueous solution of tetramethylammonium hydroxide having a concentration of 2.38% by weight, and dried to form contact holes having a diameter of 130 nm.

The thus-obtained substrate having contact holes was set in an ICP plasma etching apparatus. The inside of the apparatus was vacuumed, and perfluoro-2-pentyne as a dry etching gas, produced in Production Example 1, was introduced into the etching apparatus at a rate of 50 sccm. Etching was carried out at a plasma density of $10^{11}$ ions/cm$^3$ while the inner pressure was maintained at 0.67 Pa.

The etching rate of the silicon oxide film was 621.0 nm/min at the central portion and 564.1 nm/min at the edge portion. The etching rate of the resist film was 33.6 nm/min at the central portion and 41.5 nm/min at the edge portion. Thus, the selectivity ratio in etching was 18.5 in the central portion and 13.6 at the edge portion.

Example 2

Etching was carried out by the same procedures as those adopted in Example 1 except that perfluoro-2-butyne produced in Production Example 2 was used as a dry etching gas with all other conditions remaining the same. Etching rate and selectivity ratio were evaluated in the same manner as in Example 1.

Example 3

Etching was carried out by the same procedures as those adopted in Example 1 except that perfluoro-2-pentene produced in Production Example 3 was used as a dry etching gas with all other conditions remaining the same. Etching rate and selectivity ratio were evaluated in the same manner as in Example 1.

Example 4

Etching was carried out by the same procedures as those adopted in Example 1 except that perfluoro-1,3-butadiene having a purity of 99.9% by volume, prepared by fractionating commercially available perfluoro-1,3-butadiene (available from SynQuest Laboratories, Inc.), was used as a dry etching gas with all other conditions remaining the same. Etching rate and selectivity ratio were evaluated in the same manner as in Example 1.

Comparative Example 1

Etching was carried out by the same procedures as those adopted in Example 1 except that perfluoropropene having a purity of 99.9% by volume, prepared by fractionating commercially available perfluoropropene (available from SynQuest Laboratories, Inc.), was used as a dry etching gas with all other conditions remaining the same. Etching rate and selectivity ratio were evaluated in the same manner as in Example 1. The results are shown in Table 1.

As seen from Table 1, when perfluoropropene was used as an etching gas, a resist film for an ArF excima-laser tends to be etched, and the selectivity ratio in etching is very small.

Example 5

Ten parts of a terpolymer comprised of 2,2,2-trifluoroethyl methacrylate units, 2-ethyladamantyl methacrylate units and t-butyl methacrylate units (copolymerization ratio: 0.4:0.35:0.25 by mole; molecular weight: 8,700) and 0.15 part of triphenylsulfonium trifluoromethane sulfonate as an acid generator were dissolved in 70 parts of propylene glycol monomethyl ether acetate, and the resulting solution was filtered through a filter having a pore diameter of 100 nm to give a resist solution.

A silicon substrate of an 8 inch diameter having a coating of silicon oxide film with a thickness of 2 $\mu$m was coated with the above-mentioned resist solution by a spin-coating procedure, and then, the thus-coated substrate was pre-baked at 120°C on a hot plate to form a resist film having a thickness of 300 nm. The resist film was exposed to light through a mask pattern by an X-ray aligner. The X-ray-exposed resist film was post-baked at 130°C, and then subjected to a developing treatment at 25°C for 60 seconds with an aqueous solution of tetramethylammonium hy-

## EP 1 542 268 A1

droxide having a concentration of 2.38% by weight, and dried to form contact holes having a diameter of 100 nm.

The thus-obtained substrate having contact holes was set in an ICP plasma etching apparatus. The inside of the apparatus was vacuumed, and perfluoro-2-pentyne as a dry etching gas, produced in Example 6, mentioned below, was introduced into the etching apparatus at a rate of 50 sccm. Etching was carried out at a plasma density of $10^{11}$ ions/cm$^3$ while the inner pressure was maintained at 0.67 Pa. Etching rate and selectivity ratio were evaluated in the same manner as in Example 1.

The results obtained in Examples 1 to 5 and Comparative Example 1 are shown in Table 1.

### Table 1

| Examples | Ex. 1 | Ex. 2 | Ex. 3 | Ex. 4 | Com.Ex.1 | Ex. 5 |
|---|---|---|---|---|---|---|
| Resist | for ArF laser | | | | | for X-ray |
| Dry etching gas | A | B | C | D | E | A |
| Etching rate | | | | | | |
| Silicon oxide film | | | | | | |
|   Central portion | 621.0 | 593.5 | 518.4 | 521.2 | 500.9 | 550.1 |
|   Edge portion | 564.1 | 520.4 | 410.2 | 415.4 | 468.9 | 516.2 |
| Resist | | | | | | |
|   Central portion | 33.6 | 42.1 | 43.9 | 43.4 | 156.5 | 44.7 |
|   Edge portion | 41.5 | 56.6 | 65.1 | 63.9 | 426.3 | 51.1 |
| Selectivity | | | | | | |
|   Central portion | 18.5 | 14.1 | 11.8 | 12.0 | 3.2 | 12.3 |
|   Edge portion | 13.6 | 9.2 | 6.3 | 6.5 | 1.1 | 10.1 |

Dry etching gas    A: Perfluoro−2−pentyne
                          B: Perfluoro−2−butyne
                          C: Perfluoro−2−pentene
                          D: Perfluoro−1,3−butadiene
                          E: Perfluoropropene

Etching rate, unit: nm/min
Selectivity ratio: (etching rate for silicon oxide film)/(etching rate for resist)

As seen from Table 1, in the case when a resist pattern is formed by irradiation with an ArF excima laser and the resist is dry-etched using perfluor-2-pentyne, perfluoro-2-butyne, perfluoro-2-pentene or perfluoro-1,3-butadiene (Examples 1 to 4), the etching rate for silicon oxide film is high and the selectivity ratio is large, as compared with the case when dry etching is carried out by using perfluoropropene as a dry etching gas (Comparative Example 1). In the case when a resist pattern is formed by X-ray irradiation, and the resist is dry-etched using perfluor-2-pentyne (Example 5), the etching rate for silicon oxide film is high and the selectivity ratio is large.

Example 6 (Synthesis of perfluoro-2-pentyne)

A round glass flask equipped with a cooling tube was charged with 140 parts of dimethylformamide. The flask was cooled to -50°C, and pentafluoropropionaldehyde was blown into the dimethylformamide to prepare a solution of pentafluoropropionaldehyde in dimethylformamide. The pentafluoropropionaldehyde used was prepared by heating 12.8 parts of pentafluoropropionaldehyde hydrate (available from Central Yakuhin K.K.) together with phosphorus(V) oxide whereby the pentafluoropropionaldehyde hydrate was dehydrated. To the solution of pentafluoropropionaldehyde, 37.5 parts of 1,1,1-trichloro-2,2,2-trifluoroethane (available from Tokyo Kasei Kogyou K.K.) and 39.2 parts of zinc powder, previously activated with acetic anhydride, were added at 0°C. The thus-obtained reaction mixture was further stirred at 0°C for 30 minutes, and then, a reaction was carried out at 50°C for 4 hours. Then the reaction mixture was cooled to room temperature, and 300 parts of water was added. The aqueous mixture was extracted with 107 parts of diethyl ether. This extraction was repeated twice using the same amount of diethyl ether.

The thus-obtained liquid extract was dried over sodium sulfate. The dried product was distilled to remove ether to give 36 parts of a crude product. GC-MS analysis of the crude product revealed that 2-chloro-1,1,1,4,4,5,5,5-octafluoro-2-pentene was produced. A Hastelloy autoclave was charged with 23.9 parts of commercially available palletized potassium hydroxide having a purity of 85% by weight and 36 parts of the above-mentioned 2-chloro-1,1,1,4,4,5,5,5-octafluoro-2-pentene. The content was thoroughly stirred and a reaction was carried out at 200°C for 6 hours.

The autoclave was cooled, and the reaction mixture was distilled out. A trap for collection and a vacuum pump were connected, and the reaction mixture was collected at a subatmospheric pressure in the trap, cooled by liquid nitrogen, to give 3.7 parts of a reaction product.

The above-mentioned reaction procedures were repeated to collect the reaction products. 120 parts of the collected reaction products were distilled by the same procedures as in Production Example 1 to give 97.2 parts of perfluoro-2-pentyne fraction (boiling point: 5°C) having a purity of 99.9% by volume.

Industrial Applicability

According to the dry etching method of the present invention, a high etching rate and a high etching selectivity can be attained and a fine resist pattern can be formed with good stability on a silicon substrate, even for a resist having poor resistance to dry etching using a light source with a short wavelength of 195 nm or less.

Therefore, the dry etching method of the present invention is beneficially adopted for the production of semiconductor devices having integrated circuits of high integration and high performance such as VLSI (very large scale integrated circuit) and ULSI (ultra large scale integrated circuit).

According to the process for producing perfluoro-2-pentyne of the present invention, perfluoro-2-pentyne having a high purity can be easily produced from a readily available raw material.

**Claims**

1. A dry etching method **characterized in that** a resist film formed on a substrate is irradiated with radiation having a wavelength of not more than 195 nm to form a resist pattern having a minimum line width of not more than 200 nm, and the substrate having the resist pattern formed thereon is subjected to dry etching using a fluorine-containing compound having 4 to 6 carbon atoms and at least one unsaturated bond as an etching gas.

2. The dry etching method according to claim 1, wherein the resist film is formed from a high molecular weight compound containing 0% to 10% by weight of repeating units having an aromatic ring structure.

3. The dry etching method according to claim 1 or 2, wherein the fluorine-containing compound having 4 to 6 carbon atoms and at least one unsaturated bond is selected from perfuloro-2-butyne and perfuloro-2-pentyne.

4. The dry etching method according to claim 1 or 2, wherein the fluorine-containing compound having 4 to 6 carbon atoms is perfuloro-2-pentyne.

5. The dry etching method according to claim 1 or 2, wherein the fluorine-containing compound having 4 to 6 carbon atoms and at least one unsaturated bond is at least one kind of fluoropentene selected from 1,1,1,2,4,4,5,5,5-nonafluoro-2-pentene, 1,1,1,3,4,4,5-nonafluoro-2-pentene and perfluoro-2-pentene.

6. The dry etching method according to any one of claims 1 to 5, wherein the dry etching is carried out under irradiation with plasma having a plasma density of at least $10^{10}$ ions/cm$^3$.

7. A dry etching gas comprised of a fluorine-containing compound having 4 to 6 carbon atoms and at least one unsaturated bond, and used for dry etching for a resist film forming a resist pattern having a minimum line width of not more than 200 nm at irradiation with radiation having a wavelength of not more than 195 nm.

8. The dry etching gas according to claim 7, wherein the fluorine-containing compound having 4 to 6 carbon atoms and at least one unsaturated bond is selected from perfuloro-2-butyne and perfuloro-2-pentyne.

9. The dry etching gas according to claim 7, wherein the fluorine-containing compound having 4 to 6 carbon atoms and at least one unsaturated bond is perfuloro-2-pentyne.

**10.** The dry etching gas according to claim 7, wherein the fluorine-containing compound having 4 to 6 carbon atoms and at least one unsaturated bond is at least one kind of fluoropentene selected from 1,1,1,2,4,4,5,5,5-nonafluoro-2-pentene, 1,1,1,3,4,4,5-nonafluoro-2-pentene and perfluoro-2-pentene.

**11.** A process for producing perfluoro-2-pentyne **characterized in that** a 1,1,1-trihalo-2,2,2-trifluoroethane is allowed to react with pentafluoropropionaldehyde to give a 2-halo-1,1,1,4,4,5,5,5-octafluoro-2-pentene, and the thus-produced 2-halo-1,1,1,4,4,5,5,5-octafluoro-2-pentene is dehydrohalogenated.

**12.** The process for producing perfluoro-2-pentyne according to claim 11, wherein the 1,1,1-trihalo-2,2,2-trifluoroethane is 1,1,1-trichloro-2,2,2-trifluoroethane.

## INTERNATIONAL SEARCH REPORT

| International application No. |
| --- |
| PCT/JP03/09023 |

**A. CLASSIFICATION OF SUBJECT MATTER**
  Int.Cl$^7$  H01L21/3065

According to International Patent Classification (IPC) or to both national classification and IPC

**B. FIELDS SEARCHED**

Minimum documentation searched (classification system followed by classification symbols)
  Int.Cl$^7$  H01L21/3065

Documentation searched other than minimum documentation to the extent that such documents are included in the fields searched
  Jitsuyo Shinan Koho          1922-1996   Toroku Jitsuyo Shinan Koho   1994-2003
  Kokai Jitsuyo Shinan Koho    1971-2003   Jitsuyo Shinan Toroku Koho   1996-2003

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2000-206704 A  (Toshiba Corp.),<br>28 July, 2000 (28.07.00),<br>Par. Nos. [0014] to [0355]<br>(Family: none) | 1,2,7 |
| X | JP 2001-110790 A  (Matsushita Electric Industrial Co., Ltd.),<br>20 April, 2001 (20.04.01),<br>Par. Nos. [0016] to [0105]<br>(Family: none) | 3,6,8 |
| A | WO 00/30168 A  (APPLIED MATERIALS INC.),<br>25 February, 2000 (25.02.00),<br>Page 9, line 20 to page 30, line 29<br>& JP 2002-530863 A | 5,10 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20 October, 2003 (20.10.03) | 04 November, 2003 (04.11.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)

11

EP 1 542 268 A1

**INTERNATIONAL SEARCH REPORT**

International application No.

PCT/JP03/09023

C (Continuation).   DOCUMENTS CONSIDERED TO BE RELEVANT

| Category* | Citation of document, with indication, where appropriate, of the relevant passages | Relevant to claim No. |
|---|---|---|
| E,X | JP 2003-282540 A (Tokyo Electron Ltd.),<br>03 October, 2003 (03.10.03),<br>Par. Nos. [0010] to [0038]<br>(Family: none) | 4,8,9 |
| X | EP 755909 A (DAIKIN INDUSTRIES, LTD.),<br>29 January, 1997 (29.01.97),<br>Page 2, line 46 to page 8, line 4<br>& JP 07-278029 A         & DE 69513965 A | 11,12 |

Form PCT/ISA/210 (continuation of second sheet) (July 1998)

12

## INTERNATIONAL SEARCH REPORT

| International application No. |
| --- |
| PCT/JP03/09023 |

**Box I   Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

The invention claimed in claims 1-10 relates to a method of dry etching through exposing a resist to radiation of 195 nm or less wavelength so as to form a resist pattern of 200 nm or less minimum line width.

The invention claimed in claims 11 and 12 relates to a process for producing perfluoro-2-pentyne whose use is not limited.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐      The additional search fees were accompanied by the applicant's protest.

☐      No protest accompanied the payment of additional search fees.

Form PCT/ISA/210 (continuation of first sheet (1)) (July 1998)